Europäisches Patentamt

European Patent Office

Office européen des brevets

(19)

(11) EP 0 897 414 B1

(12) FASCICULE DE BREVET EUROPEEN

(45) Date de publication et mention
de la délivrance du brevet:
**05.09.2001 Bulletin 2001/36**

(51) Int Cl.⁷: **C09C 1/30**, C01B 33/18,
C08K 3/36, D21H 17/68,
D21H 19/40, C09D 7/12,
B01J 20/10, A61K 7/16,
A61K 7/42, C04B 14/04

(21) Numéro de dépôt: **97920804.8**

(22) Date de dépôt: **22.04.1997**

(86) Numéro de dépôt international:
**PCT/FR97/00722**

(87) Numéro de publication internationale:
**WO 97/40105 (30.10.1997 Gazette 1997/46)**

(54) **PROCEDE DE PREPARATION DE PARTICULES CREUSES DE SILICE**

VERFAHREN ZUR HERSTELLUNG VON HOHLEN KIESELSÄURETEILCHEN

METHOD FOR PREPARING HOLLOW SILICA PARTICLES

(84) Etats contractants désignés:
**BE DE ES FI FR GB NL**

(30) Priorité: **22.04.1996 FR 9605136**

(43) Date de publication de la demande:
**24.02.1999 Bulletin 1999/08**

(73) Titulaire: **RHODIA CHIMIE**
**92408 Courbevoie Cédex (FR)**

(72) Inventeur: **AMICHE, Frédéric**
**F-92420 Vaucresson (FR)**

(74) Mandataire: **Fabre, Madeleine-France et al**
**Rhodia Services,**
**Direction de la Propriété Industrielle,**
**40, rue de la Haie-Coq**
**93306 Aubervilliers Cedex (FR)**

(56) Documents cités:
**EP-A- 0 396 450          WO-A-96/11054**
**US-A- 2 885 366          US-A- 5 024 826**
**US-H- H1 447**

## Description

La présente invention a pour objet un procédé de préparation de particules creuses comprenant une écorce de silice dense, par précipitation de silice active à partir d'une solution aqueuse de silicate de métal alcalin sur un coeur constitué en un matériau autre que de la silice et élimination dudit matériau sans destruction de l'écorce de silice. Lesdites particules peuvent être utilisées comme matériaux isolants, comme charges creuses pour polymères, matériaux de construction, caoutchouc, papier, peinture, comme agents absorbants, comme abrasifs ou additifs pour dentifrice, comme supports pour l'absorption et/ou le relargage de matière active, la libération de ladite matière active étant réalisée par destruction de l'écorce de silice par rupture mécanique ou par dissolution en milieu basique de l'écorce de silice ou par diffusion, comme agents de protection solaire ou support d'agents de protection solaire ou pour la formulation sous forme solide de matières liquides.

Il est connu de préparer des particules hétérogènes constituées d'une écorce de silice dense déposée sur un coeur constitué d'une charge autre que de la silice, par précipitation lente de silice active sur ledit coeur, à partir d'une solution aqueuse de silicate de métal alcalin avec réglage du pH à l'aide d'un acide (US-A-2,885,366). Selon ce document, l'opération de précipitation doit être effectuée au sein d'un milieu de faible force ionique, avec une vitesse d'ajout de silicate inférieure à un certain paramètre S (exprimé en masse de silice à ajouter par heure, par rapport à la masse de coeur à enrober) défini par l'équation suivante

$$S = (A/200) \, 2^n$$

- $\underline{n}$ étant égal à $(T-90) / 10$
- $\underline{A}$ représentant la surface spécifique, exprimée en $m^2/g$, du support à enrober
- et T la température en °C,

ce afin d'éviter la formation de nuclei de particules denses de silice.

De ce fait, l'opération de précipitation de la silice active est longue ; ainsi, le dépot de l'ordre de 20 parties en masse de silice sur 100 parties en masse de carbonate de calcium à une température de l'ordre de 80 à 90°C, nécessite entre 3 et 5 heures de réaction de précipitation.

Lorsque que le coeur desdites particules de silice ainsi obtenues est constitué d'un composé sensible aux acides, des particules creuses de silice peuvent alors être obtenues par élimination du coeur par attaque acide (US-A-5,024,826 et H 1447).

La demanderesse a trouvé un nouveau procédé permettant de préparer des particules creuses de silice dense par précipitation rapide de la silice active sur un coeur constitué d'un matériau différent de la silice, sans risque de formation de nuclei de particules de silice, puis élimination dudit matériau sans destruction de l'écorce de silice active dense.

On entend, d'une manière simplifiée, comme "dense", une écorce de silice formée d'une couche continue constituée d'un réseau de silice, en opposition à une couche constituée d'un assemblage poreux de particules élémentaires de silice.

La présente invention consiste donc en un procédé de préparation de particules creuses comprenant une écorce de silice dense, par précipitation de silice active à partir d'une solution aqueuse de silicate de métal alcalin M, de rapport $SiO_2/Na_2O$ de 2 au moins, de préférence de l'ordre de 2,5 à 4, avec réglage du pH à l'aide d'un agent acidifiant, sur un support en un matériau autre que la silice, séparation de la bouillie de silice formée et séchage de la suspension de silice récupérée, avec élimination du support sans dissolution ni destruction de l'écorce de silice active, l'opération de formation de bouillie de silice par précipitation étant réalisée selon les étapes suivantes :

* une première étape consistant à mettre en oeuvre un pied de cuve initial de pH de l'ordre de 8 à 10, comprenant

  - de l'eau,
  - au moins un support organique ou inorganique différent de la silice, support insoluble dans l'eau dans les conditions de pH et de température de l'opération de formation de bouillie, mais susceptible d'être au moins partiellement éliminable sans dissolution ou destruction de l'écorce de silice, lors de l'opération ultérieure d'élimination,
  - éventuellement un agent basique ou tampon,

  à une température de l'ordre de 80 à 98°C ;
* une deuxième étape consistant à introduire dans ledit pied de cuve,

  - le silicate de métal alcalin sous forme d'une solution aqueuse
  - et l'agent acidifiant ;

  et ce jusqu'à ce que la quantité désirée de silice soit formée,
  le milieu réactionnel, à cette deuxième étape, présentant un pH sensiblement constant de l'ordre de 8 à 10 et étant maintenu à une température de l'ordre de 80 à 98°C,
  ledit procédé étant caractérisé en ce que
* le pied de cuve initial mis en oeuvre à la première étape comprend un sel électrolyte du groupe des métaux alcalins, la quantité d'électrolyte présente étant d'au moins environ 0,4 mole, de préférence de l'ordre de 0,4 à 1,5 mole d'ion métal alcalin par litre de pied de cuve,

* la solution de silicate de métal alcalin mise en oeuvre à la deuxième étape contient au moins environ 100 grammes de $SiO_2$ / litre, de préférence de l'ordre de 100 à 330 grammes de $SiO_2$ / litre,
* et en ce que ladite deuxième étape est réalisée dans des conditions telles que la cinétique C de formation de silice active, exprimée en grammes de silice / heure / gramme de support, corresponde à une valeur

$$C \geq 3 \, (A/200) \, 2^n,$$

de préférence $C \geq 4 \, (A/200) \, 2^n$
et tout particulièrement $C \geq 6 \, (A/200) \, 2^n$

- $\underline{n}$ étant égal à $(T-90) /10$
- $\underline{A}$ représentant la surface spécifique, exprimée en $m^2/g$, du support à enrober
- et T la température en $°C$.

Le choix du silicate et de l'agent acidifiant pour réaliser le procédé de l'invention, se fait d'une manière bien connue en soi.

Le silicate de métal alcalin est avantageusement un silicate de sodium ou de potassium. On peut citer tout particulièrement les silicates de sodium.

On utilise généralement comme agent acidifiant à la deuxième étape de formation de bouillie, un acide minéral tel que l'acide sulfurique, l'acide nitrique, l'acide chlorhydrique ou un acide organique comme l'acide acétique, l'acide formique ou l'acide carbonique. D'une manière préférentielle, il s'agit de l'acide sulfurique. Ce dernier peut être mis en oeuvre sous forme diluée ou concentré, de préférence sous forme d'une solution aqueuse présentant une concentration de l'ordre de 60 à 400 g/l. L'acide carbonique est de préférence mis en oeuvre sous forme gazeuse.

Parmi les matériaux pouvant être mis en oeuvre comme support, pour la réalisation du procédé de l'invention, on peut mentionner tout composé inerte vis-à-vis de la silice active (silice hydroxylée), insoluble dans l'eau dans les conditions de pH et de température de l'opération de formation de bouillie, de forme quelconque (sphérique, aciculaire, ...) solide ou liquide, minéral ou organique, susceptible d'être éliminé des particules le renfermant par un traitement fonction de sa nature chimique et physique. D'une manière préférentielle, ledit support est sous forme solide.

On entend par "composé inerte vis-à-vis de la silice" tout composé restant stable dans les conditions de précipitation de la silice.

On entend par "composé insoluble dans l'eau", tout composé présentant une solubilité dans l'eau inférieure à environ 0,5% en poids à 25°C.

En ce qui concerne l'opération d'élimination du matériau constituant le support (coeur), il peut s'agir, selon la nature dudit matériau, d'un traitement par dissolution (à l'aide d'un acide ou d'un solvant apolaire) pour les solides, suivi d'une opération de séparation ; d'un traitement thermique quel que soit le matériau ; ou d'une simple opération de séparation lorsqu'il s'agit d'un liquide.

Quelle que soit la nature du matériau (solide, liquide, organique, minéral), celui-ci peut être éliminable par traitement thermique, à une température au moins égale à celle de vaporisation ou de décomposition dudit matériau, mais n'endommageant pas l'écorce de silice.

Parmi les matériaux solides, peuvent être mis en oeuvre ceux éliminables par dissolution à l'aide d'un acide minéral ou organique à un pH inférieur à 8, de préférence de l'ordre de 2 à 7 ou d'un solvant apolaire, cette opération de dissolution étant suivie d'une séparation par exemple par centrifugation, filtration, distillation, évaporation, dialyse, osmose ...

Parmi les matériaux solides susceptibles d'être éliminés par dissolution à l'aide d'un acide, de préférence en solution aqueuse, on peut citer les sels inorganiques comme le carbonate de calcium, le carbonate de baryum ..., les métaux comme le cuivre ..., les polymères organiques acidosolubles comme les polymères aminés (comme ceux dérivés de la vinylpyridine), les polysaccharides réticulés ...La nature de l'acide à mettre en oeuvre est bien entendu fonction de la nature chimique dudit matériau.

Parmi les matériaux solides susceptibles d'être dissous par des solvants apolaires, on peut citer les polymères organiques non alcalisolubles, tels que notamment les polystyrènes, les esters polyacryliques, polyméthacryliques, les polyéthylènes, les polyamides, les polyesters ...La nature du solvant apolaire à mettre en oeuvre est bien entendu fonction de la nature chimique dudit matériau. Les solvants chlorés (dichlorométhane ...), le tétrahydrofurane ... sont généralement bien adaptés.

En ce qui concerne les matériaux liquides, peuvent être mis en oeuvre ceux éliminables par une simple opération de séparation comme la centrifugation, filtration, distillation, évaporation, dialyse, osmose ...

Parmi les matériaux susceptibles d'être ainsi séparés, on peut mentionner, les huiles végétales, les huiles minérales, l'huile de vaseline, les huiles silicones ...

Le support mis en oeuvre peut être de taille quelconque selon le volume de vide recherché, par exemple de l'ordre de 20nm à 30μm, de préférence de l'ordre de 50nm à 20μm.

Parmi les électrolytes pouvant être mis en oeuvre pour préparer le pied de cuve, on peut citer notamment le sel du métal de silicate de départ et de l'agent acidifiant ; de préférence il s'agit de sulfate de sodium ; toutefois du chlorure, nitrate ou hydrogénocarbonate de sodium peut être préféré si la présence d'ions sulfates résiduels n'est pas désirée.

La première étape de l'opération de formation de bouillie consiste à préparer le pied de cuve initial.

Si le support mis en oeuvre est un matériau solide, celui-ci peut être introduit tel quel ou de préférence sous forme d'une dispersion aqueuse. S'il s'agit d'un liquide, celui-ci est de préférence introduit sous forme d'un émulsion aqueuse.

La quantité de support pouvant être mise en oeuvre est telle que le pied de cuve formé renferme de l'ordre d'au moins 10% de son poids de support solide, ou de l'ordre d'au moins 10% de son volume de support liquide ; ledit pied de cuve peut généralement contenir jusqu'à 50% de son poids ou de son volume de support solide ou liquide.

Un agent basique ou tampon peut être mis en oeuvre dans le pied de cuve initial pour assurer un pH dudit pied de cuve de l'ordre de 8 à 10.

On peut citer comme agent basique ou tampon, les hydroxydes de métaux alcalins comme l'hydroxyde de sodium, les silicates de métaux alcalins en solution, les phosphates de métaux alcalins, les hydrogénocarbonates de métaux alcalins ...

Le pied de cuve obtenu est porté à une température de l'ordre 80 à 98°C.

La deuxième étape de l'opération de formation de bouillie par précipitation, consiste à ajouter au pied de cuve maintenu sous agitation, la solution de silicate et l'agent acidifiant simultanément.

Les quantités respectives de silicate de métal alcalin et d'agent acidifiant sont choisies de manière à obtenir la cinétique C de formation de silice active mentionnée ci-dessus et de manière à maintenir le pH du milieu réactionnel à une valeur sensiblement constante de l'ordre de 8 à 10 pendant toute l'introduction des deux réactifs. Ces deux réactifs sont introduits en maintenant le milieu à une température de l'ordre de 80 à 98°C.

On arrête l'introduction de la solution de silicate lorsque la quantité désirée de silice est formée. La quantité minimum recherchée de silice est celle correspondant à un dépôt de l'ordre de 1 à 150 parties en poids de $SiO_2$ pour 100 parties en poids de support.

Cette deuxième étape dure généralement de l'ordre de 30 minutes à 2 heures.

Le pH du milieu obtenu en fin de deuxième étape, après arrêt de l'introduction des réactifs, est ensuite amené, si nécessaire, à une valeur inférieure à 7, de préférence de l'ordre de 4 à 5.

Le milieu obtenu en fin de deuxième étape, après arrêt de l'introduction des réactifs, est éventuellement laissé mûrir pendant environ 10 à 30 minutes dans les mêmes conditions de température. Cette opération éventuelle de mûrissement peut être réalisée soit avant soit après avoir amené le pH du milieu à une valeur inférieure à 7, de préférence de l'ordre de 4 à 5, si cette rectification de pH est nécessaire.

On obtient à l'issue des opérations ci-dessus décrites de formation de bouillie, une bouillie de silice, qui est ensuite séparée (séparation liquide-solide); cette opération consiste généralement en une filtration (par exemple décantation, utilisation d'un filtre rotatif sous vide), suivie d'un lavage à l'eau et éventuellement à l'alcool et à l'éther.

La suspension de silice ainsi récupérée (gâteau de filtration) est ensuite séchée (étuve, four, atomisation, vide).

Les particules ainsi obtenues peuvent présenter une épaisseur d'écorce de silice de l'ordre de 2 à 200 nm, de préférence de l'ordre de 2 à 50nm, pour une taille de coeur de support de l'ordre de 20nm à 30μm, de préférence de l'ordre de 50nm à 20μm.

Comme mentionné plus haut, l'opération d'élimination du matériau constituant le support peut être réalisée par différents types de traitement, thermique quelle que soit la nature du support, dissolution suivie d'une séparation s'il s'agit d'un solide ou simple séparation s'il s'agit d'un liquide.

Lorsqu'il s'agit d'un traitement thermique, celui-ci est réalisé à une température au moins égale à celle nécessaire pour vaporiser ou calciner le matériau constituant le support ; il peut être réalisé sur les particules obtenues après lavage du gâteau de filtration ou sur les particules séchées.

Lorsqu'il s'agit d'un traitement par dissolution acide, celui-ci est réalisé à un pH inférieur à 8, de préférence de l'ordre de 2 à 7, avant ou après séparation de la bouillie de particules comprenant une écorce de silice.

Ainsi ledit traitement peut être réalisé tout aussi bien :

- à la fin de la deuxième étape de l'opération de formation de bouillie, sur la bouillie obtenue après arrêt de l'introduction des réactifs et éventuel mûrissement du milieu réactionnel
- qu'à la fin de la deuxième étape de l'opération de formation de bouillie par précipitation, pendant le mûrissement du milieu réactionnel
- qu'après séparation de la bouillie, sur le gâteau de filtration, avant ou après lavage
- ou qu'après séchage des particules et redispersion desdites particules dans l'eau.

Les acides pouvant être mis en oeuvre pour réaliser le traitement chimique acide, sont choisis parmi ceux susceptibles de dissoudre le matériau constituant le coeur.

Ainsi lorsqu'il s'agit de carbonate de calcium, les acides préférentiellement mis en oeuvre sont les acides forts, notamment les acides chlorhydrique et nitrique, sous forme de solutions aqueuses.

L'ajout d'acide est de préférence réalisé progressivement, et ce jusqu'à ce que le pH se stabilise à une valeur inférieure à 8, de préférence de l'ordre de 2 à 4.

Les particules traitées sont ensuite récupérées par centrifugation, filtration, distillation, évaporation, dialyse, osmose ..., lavées à l'eau et séchées.

Lorsqu'il s'agit d'un traitement de dissolution par un solvant apolaire, celui-ci est réalisé après séchage des particules, redispersion desdites particules dans ledit solvant puis séparation par centrifugation, filtration, distillation, évaporation, dialyse, osmose ..., lavage à l'eau et séchage.

Selon une variante de réalisation de l'invention, l'écorce de silice contient en outre des traces de cations polyvalents, tels que $Mg^{2+}$, $Ca^{2+}$, $Ba^{2+}$, $Pb^{2+}$ introduits de préférence sous forme de solution aqueuse soit au cours de l'opération de formation de bouillie à la première étape dans le pied de cuve ou à la deuxième étape pendant l'ajout simultané des réactifs. Cette présence de cations est tout particulièrement intéressante pour apporter de la microporosité à l'écorce de silice dense, par attaque acide pour éliminer le coeur.

Le procédé faisant l'objet de l'invention, est bien adapté à la préparation de particules creuses de silice dense, éventuellement en outre microporeuse, présentant :

- une surface BET de l'ordre de 15 à 800 $m^2/g$
- une prise d'huile DOP supérieure à 500ml/100g de silice
- et une épaisseur d'écorce de l'ordre de 2 à 200 nm, de préférence de l'ordre de 2 à 50nm.

Lesdites particules présentent une granulométrie fonction de la taille du support de départ ; d'une manière préférentielle, celle-ci peut être de l'ordre de plus de 20nm à 30μm, de préférence de l'ordre de 50nm à 20 μm.

La surface spécifique BET est déterminée selon la méthode de BRUNAUER - EMET - TELLER décrite dans "The journal of the American Chemical Society", Vol. 60, page 309, février 1938 et correspondant à la norme NFT 45007 (novembre 1987).

La prise d'huile DOP est déterminée selon la norme ISO 787/5 en mettant en oeuvre du dioctylphtalate.

L'épaisseur de l'écorce est déterminée par microscopie électronique.

Les particules creuses obtenues selon le procédé de l'invention, peuvent être utilisées

- comme matériau ou constituant de matériau isolant thermique ou phonique
- comme charges creuses pour polymères, matériaux de construction, caoutchouc, papier (papier jet d'encre), peinture ...
- comme agent absorbant (traitement d'effluents, papier absorbant ...)
- comme abrasif pour dentifrice.
- comme support pour l'absorption et/ou pour le relargage contrôlé de matières actives pouvant être mises sur support telles quelles à l'état liquide, ou sous forme liquide en solution ou à l'état fondu, le relargage de la matière active étant alors réalisé par destruction de l'écorce de silice, par exemple par dissolution dans un milieu fortement basique ou par action mécanique, ou par diffusion

Ainsi, elles peuvent être utilisées pour l'absorption

- d'agents accélérateurs de prise ou d'agents viscosants pour bétons et matériaux de construction, avec relargage contrôlé du principe actif

  . d'agents oxydants utilisables dans les opérations de fracturation dans le domaine pétrolier
  . et le relargage contrôlé de principes actifs pharmaceutiques, agrochimiques, alimentaires, cosmétiques, d'arômes, de parfums
  . de bactéricides pour le nettoyage de surfaces dures en détergence ménagère ou industrielle
  . d'agents émollients ou hydratants pour l'hygiène corporelle
  . d'enzyme (application en détergence ménagère)

- comme agent de protection solaire ou comme support d'agents de protection solaire (anti U-V)
- comme abrasif ou additif pour dentifrice avec relargage contrôlé d'arômes ou de principes actifs thérapeutiques (dérivés fluorés, bactéricides ...) absorbés ; lorsque la matière active est un arôme, celle-ci, mise ainsi sur support, peut avoir une réactivité et/ou une adsorption limitée avec et/ou sur les abrasifs classiques présentant généralement des surfaces spécifiques élevées
- pour la formulation sous forme solide de produits classiquement utilisés dans les formulations liquides, produits tels que les matières liquides biologiquement actives pour application phytosanitaire ou thérapeutique et tels que les huiles ou dérivés d'huiles organiques, minérales, végétales, silicones, pour la formulation de solides tels que des savons.

Un deuxième objet de l'invention consiste en des particules de silice dense creuses cassantes présentant

- une surface BET de l'ordre de 15 à 800 $m^2/g$
- une prise d'huile DOP supérieure à 500 ml/100g de silice
- une épaisseur d'écorce de l'ordre de 2 à moins de 10 nm.
- et une granulométrie supérieure à 15μm environ.

Ces particules peuvent être utilisées comme support de matières actives pour la protection et/ou la libération de matières actives telles que les arômes, dérivés d'arômes, parfums, émollients, agents humectants, hydratants, conditionants ..., la libération de la matière active étant réalisée par rupture mécanique de l'écorce de silice par simple contrainte mécanique (écrasement par passage dans un atomiseur, écrasement par étalement manuel sur la peau ...)
Lesdites matières actives peuvent être absorbées par simple mise en contact sous forme liquide, [soit telles quelles ou fondues (quand il s'agit de solides, de cires ou de gels), soit en solution ou dispersion dans un véhicule], avec lesdites particules creuses de silice dense cassantes.

Les exemples suivants sont donnés à titre illustratif.

## Exemple 1

Préparation de particules constituées d'un coeur de carbonate de calcium et d'une écorce de silice

On prépare un pied de cuve, par introduction dans un réacteur de 15 litres, de 5 litres d'eau, de 0,68 mole/litre de pied de cuve, de sodium sous forme de chlorure de sodium, de 1150 g de carbonate de calcium précipité présentant une granulométrie de $4\mu m$, une surface spécifique BET de 16 $m^2/g$ et de silicate de sodium de rapport $SiO_2/Na_2O$ de 3,5 (solution aqueuse à 130g de $SiO_2$ par litre) en quantité correspondant à une concentration de 3 g de $SiO_2$ par litre de pied de cuve. Le pied de cuve de pH 9, est porté à 90°C et maintenu sous agitation.

On introduit ensuite simultanément

- une solution aqueuse de silicate de sodium de rapport $SiO_2/Na_2O$ de 3,5 dont la concentration est de 130g de $SiO_2$ par litre de solution,
- et une solution aqueuse d'acide sulfurique contenant 80g d'acide par litre,

de façon à former 230g de silice en 30 minutes.

Après mûrissement pendant 30 minutes, la bouillie obtenue est filtrée ; le gâteau de filtration est lavé à l'eau puis séché en étuve à 80°C.

L'analyse du produit par microscopie électronique (MET) montre que l'épaisseur de la couche de silice déposée est de l'ordre de 5nm.

La surface BET des particules est de 21 $m^2/g$.

La cinétique d'ajout du silicate de sodium a été de 0,4g $(SiO_2)/h/g(CaCO_3)$, contre 0,08g$(SiO_2)/h/g(CaCO_3)$ selon l'art antérieur (US-A-2,885,366).

Elimination du coeur

300 g des particules sèches obtenues ci-dessus sont redispersées dans 2,7 litres d'eau. De l'acide chlorhydrique concentré (8,5 M) est ajouté de façon à descendre le pH jusqu'à 2 ; l'addition d'acide se poursuit pendant 30 minutes jusqu'à stabilisation à ce pH.

Le produit obtenu est ensuite filtré, lavé abondamment et séché à l'étuve à 80°C.

Les particules creuses obtenues présentent les caractéristiques suivantes :

- une surface BET de 207 $m^2/g$
- une prise d'huile DOP de 612 ml/100g de silice
- une épaisseur d'écorce de l'ordre de 5nm

## Exemple 2

On disperse sous faible cisaillement 50g de silice creuse préparée à l'exemple 1, dans 500ml de cyclométhicone (huile silicone volatile MIRASIL CM4 commercialisée par RHONE-POULENC).

Le mélange est laissé sous faible cisaillement, à température ambiante pendant 10 minutes. La quantité de cyclométhicone absorbée est mesurée par augmentation de la masse des particules sèches obtenues. On constate que la silice absorbe 5 fois sa masse en huile silicone.

## Exemple 3

On disperse sous faible cisaillement 50g de silice creuse préparée à l'exemple 1, dans 500ml d'arôme menthe HERBAL.

Le mélange est laissé sous faible cisaillement, à température ambiante pendant 10 minutes. La quantité d'arôme absorbée est mesurée par augmentation de la masse des particules sèches obtenues. On constate que la silice absorbe 5 fois sa masse en arôme.

## Exemple 4

On disperse sous faible cisaillement 50g de silice creuse préparée à l'exemple 1, dans 500ml d'une solution de concentré parfumant à 15% dans de la cyclométhicone.

Le mélange est laissé sous faible cisaillement, à température ambiante pendant 10 minutes. La quantité de solution de concentré parfumant absorbée est mesurée par augmentation de la masse des particules sèches obtenues. On constate que la silice absorbe 5 fois sa masse en concentré parfumant.

## Exemple 5

Préparation de particules constituées d'un coeur de carbonate de calcium et d'une écorce de silice

On prépare un pied de cuve, par introduction dans un réacteur de 15 litres,

- de 5 litres d'une dispersion aqueuse contenant 230g/l de carbonate de calcium précipité présentant un diamètre de $17\mu m$ et une surface spécifique BET de 4$m^2/g$,
- de sulfate de sodium en quantité correspondant à 0,43 mole de sodium par litre de pied de cuve,
- et de 124g de solution aqueuse, à 130g de $SiO_2$ par litre, de silicate de sodium de rapport $SiO_2/Na_2O$ de 3,5, pour obtenir un pH de 9 du pied de cuve.

Le pied de cuve est porté à 90°C et maintenu sous agitation.

On introduit ensuite simultanément

- une solution aqueuse de silicate de sodium de rapport $SiO_2/Na_2O$ de 3,5 dont la concentration est de 130g de $SiO_2$ par litre de solution,
- et du $CO_2$ gazeux

de façon à former 57,5g de silice en 45 minutes.

Après mûrissement pendant 30 minutes, la bouillie obtenue est filtrée ; le gâteau de filtration est lavé à l'eau puis séché en étuve à 80°C.

L'analyse du produit par microscopie électronique (MET) montre que l'épaisseur de la couche de silice déposée est de l'ordre de 5nm.

La surface BET des particules est de 4,2 m$^2$/g.

La cinétique d'ajout du silicate de sodium a été de 0,07g $(SiO_2)$/h/g$(CaCO_3)$, contre 0,02g$(SiO_2)$/h/g$(CaCO_3)$ selon l'art antérieur (US-A-2,885,366).

<u>Elimination du coeur</u>

300 g des particules sèches obtenues ci-dessus sont redispersées dans 2,7 litres d'eau.

De l'acide chlorhydrique concentré (8,5 M) est ajouté de façon à descendre le pH jusqu'à 2 ; l'addition d'acide se poursuit pendant 30 minutes jusqu'à stabilisation à ce pH.

Le produit obtenu est ensuite filtré, lavé abondamment à l'eau, puis à l'éthanol et enfin à l'éther et séché sous vide.

Les particules creuses obtenues présentent les caractéristiques suivantes :

- une surface BET de 198 m$^2$/g
- une prise d'huile DOP de 1740 ml/100g de silice
- une épaisseur d'écorce de l'ordre de 5nm

**Revendications**

1. Procédé de préparation de particules creuses comprenant une écorce de silice dense, par précipitation de silice active à partir d'une solution aqueuse de silicate de métal alcalin M, de rapport $SiO_2/Na_2O$ de 2 au moins, de préférence de l'ordre de 2,5 à 4, avec réglage du pH à l'aide d'un agent acidifiant, sur un support en un matériau autre que la silice, séparation de la bouillie de silice formée et séchage de la suspension de silice récupérée, avec élimination du support sans dissolution ni destruction de l'écorce de silice active, l'opération de formation de bouillie de silice par précipitation étant réalisée selon les étapes suivantes :

   * une première étape consistant à mettre en oeuvre un pied de cuve initial de pH de l'ordre de 8 à 10, comprenant

      . de l'eau,
      . au moins un support organique ou inorganique différent de la silice, support insoluble dans l'eau dans les conditions de pH et de température de l'opération de formation de bouillie, mais susceptible d'être au moins partiellement éliminable sans dissolution ou destruction de l'écorce de silice, lors de l'opération ultérieure d'élimination,
      . éventuellement un agent basique ou tampon,

   à une température de l'ordre de 80 à 98°C ;
   * une deuxième étape consistant à introduire dans ledit pied de cuve,

      . le silicate de métal alcalin sous forme d'une solution aqueuse
      . et l'agent acidifiant ;

   et ce jusqu'à ce que la quantité désirée de silice soit formée,
   le milieu réactionnel, à cette deuxième étape, présentant un pH sensiblement constant de l'ordre de 8 à 10 et étant maintenu à une température de l'ordre de 80 à 98°C,
   ledit procédé étant **caractérisé en ce que**
   * le pied de cuve initial mis en oeuvre à la première étape comprend un sel électrolyte du groupe des métaux alcalins, la quantité d'électrolyte présente étant d'au moins environ 0,4 mole, de préférence de l'ordre de 0,4 à 1,5 mole d'ion métal alcalin par litre de pied de cuve,
   * la solution de silicate de métal alcalin mise en oeuvre à la deuxième étape contient au moins environ 100 grammes de $SiO_2$ / litre, de préférence de l'ordre de 100 à 330 grammes de $SiO_2$ / litre,
   * et en ce que ladite deuxième étape est réalisée dans des conditions telles que la cinétique C de formation de silice active, exprimée en grammes de silice / heure / gramme de support, corresponde à une valeur

   $$C \geq 3 \ (A/200) \ 2^n,$$

   de préférence $C \geq 4 \ (A/200) \ 2^n$
   et tout particulièrement $C \geq 6 \ (A/200) \ 2^n$

      . $n$ étant égal à $(T-90)/10$
      . $A$ représentant la surface spécifique, exprimée en m$^2$/g, du support à enrober
      . et T la température en °C.

2. Procédé selon la revendication 1) **caractérisé en ce que** le silicate de métal alcalin est un silicate de sodium ou de potassium.

3. Procédé selon la revendication 1) ou 2) **caractérisé**

**en ce que** l'agent acidifiant est un acide minéral ou organique

**4.** Procédé selon la revendication 3) **caractérisé en ce que** l'agent acidifiant est de l'acide sulfurique, nitrique, chlorhydrique, acétique, formique ou carbonique.

**5.** Procédé selon la revendication 4) **caractérisé en ce que** l'agent acidifiant est de l'acide sulfurique, sous forme d'une solution aqueuse présentant une concentration de l'ordre de 60 à 400 g/l.

**6.** Procédé selon l'une quelconque des revendications précédentes, **caractérisé en ce que** le matériau constituant le support, est choisi parmi les matériaux susceptibles d'être éliminés par l'une des opérations suivantes, traitement thermique pour les matériaux solides ou liquides, traitement par dissolution à l'aide d'un acide minéral ou organique à un pH inférieur à 8, de préférence de l'ordre de 2 à 7 ou à l'aide d'un solvant apolaire pour les matériaux solides ou séparation pour les matériaux liquides, et en ce que ledit matériau est éliminé par mise en oeuvre de l'opération correspondante.

**7.** Procédé selon la revendication 6) **caractérisé en ce que** les matériaux solides susceptibles d'être éliminés par dissolution à l'aide d'un acide, sont choisis parmi les sels inorganiques, les métaux, les polymères organiques, les polysaccharides réticulés solubles à un pH inférieur à 8, de préférence de l'ordre de 2 à 7.

**8.** Procédé selon la revendication 7) **caractérisé en ce que** ledit matériau est du carbonate de calcium.

**9.** Procédé selon la revendication 6) **caractérisé en ce que** les matériaux solides susceptibles d'être éliminés par dissolution à l'aide d'un solvant apolaire sont choisis parmi les polymères organiques non alcalisolubles.

**10.** Procédé selon la revendication 6) **caractérisé en ce que** les matériaux liquides susceptibles d'être éliminés par séparation sont choisis parmi les huiles végétales, les huiles minérales, l'huile de vaseline, les huiles silicones.

**11.** Procédé selon la revendication 10) **caractérisé en ce que** l'opération de séparation est une opération de centrifugation, filtration, distillation, évaporation, dialyse ou osmose.

**12.** Procédé selon l'une quelconque des revendications précédentes, **caractérisé en ce que** le matériau autre que la silice présente une taille de l'ordre de 20nm à 30μm, de préférence de l'ordre de 50nm à 20μm.

**13.** Procédé selon l'une quelconque des revendications précédentes, **caractérisé en ce que** l'électrolyte est du sulfate, chlorure, nitrate ou hydrogénocarbonate de sodium.

**14.** Procédé selon l'une quelconque des revendications précédentes, **caractérisé en ce que** le support est mis en oeuvre sous forme d'une dispersion aqueuse lorsqu'il s'agit d'un matériau solide, ou sous forme d'un émulsion aqueuse s'il s'agit d'un liquide.

**15.** Procédé selon l'une quelconque des revendications précédentes, **caractérisé en ce que** la quantité de support pouvant être mise en oeuvre est telle que le pied de cuve formé contienne de l'ordre d'au moins 10% de son poids de support solide, ou de l'ordre d'au moins 10% de son volume de support sous forme liquide.

**16.** Procédé selon la revendication 15), **caractérisé en ce que** le pied de cuve contient jusqu'à 50% de son poids ou de son volume de support solide ou liquide.

**17.** Procédé selon l'une quelconque des revendications précédentes, **caractérisé en ce que** la deuxième étape de formation de bouillie est réalisée par introduction simultanée de silicate de métal alcalin et d'agent acidifiant, jusqu'à la formation d'au moins 1 à 150 parties en poids de $SiO_2$ pour 100 parties en poids de support.

**18.** Utilisation des particules creuses de silice obtenues selon le procédé faisant l'objet de l'une quelconque des revendications précédentes, comme matériaux ou constituants de matériaux isolants thermiques ou phoniques, comme charges creuses pour polymères, matériaux de construction, caoutchouc, papier, peinture, comme agents absorbants, comme abrasifs ou additifs pour dentifrice, comme supports pour l'absorption et/ou le relargage de matière active, la libération de ladite matière active étant réalisée par destruction de l'écorce de silice par rupture mécanique ou par dissolution en milieu basique de l'écorce de silice ou par diffusion, comme agents de protection solaire ou support d'agents de protection solaire ou pour la formulation sous forme solide de matières liquides.

**19.** Particules creuses cassantes de silice dense obtenues selon le procédé faisant l'objet de l'une quelconque des revendications 1) à 17), **caractérisées en ce qu'**elles présentent

- une surface BET de l'ordre de 15 à 800 $m^2$/g
- une prise d'huile DOP supérieure à 500 ml/100g de silice
- et une épaisseur d'écorce de l'ordre de 2 à moins de 10 nm
- et une granulométrie supérieure à 15μm environ.

**Patentansprüche**

1. Verfahren zur Herstellung von hohlen Teilchen, die eine Schale aus dichtem Siliciumdioxid umfassen, durch Fällung von aktivem Siliciumdioxid ausgehend von einer wäßrigen Lösung eines Silicates von Alkalimetall M, wobei das Verhältnis $SiO_2/Na_2O$ mindestens 2 beträgt und vorzugsweise in der Größenordnung von 2,5 bis 4 liegt, mit Regelung des pH-Wertes mit Hilfe eines Ansäuerungsmittels, auf einem Träger, der aus einem anderen Material besteht als Siliciumdioxid, Abtrennen der Aufschlämmung aus gebildetem Siliciumdioxid und Trocknen der Suspension von gewonnenem Siliciumdioxid, mit Entfernung des Trägers ohne Auflösung noch Zerstörung der Schale aus aktivem Siliciumdioxid, wobei die Operation der Bildung der Siliciumdioxid-Aufschlämmung durch Fällung gemäß der folgenden Stufen realisiert wird:

   * eine erste Stufe besteht darin, einen Ausgangsansatz mit einem pH-Wert in der Größenordnung von 8 bis 10, umfassend

      . Wasser,
      . mindestens einen organischen oder anorganischen Träger, der von Siliciumdioxid verschieden ist, wobei der Träger unter den Arbeitsbedingungen von pH und Temperatur zur Bildung der Aufschlämmung in Wasser unlöslich aber geeignet ist, mindestens teilweise ohne Auflösung oder Zerstörung der Schale aus Siliciumdioxid bei der späteren Operation der Entfernung wieder entfernt zu werden,
      . gegebenenfalls ein basisches Mittel oder einen Puffer, bei einer Temperatur in der Größenordnung von 80 °C bis 98 °C einzusetzen,

   * eine zweite Stufe besteht darin, in den genannten Ausgangsansatz einzutragen

      . das Alkalimetallsilicat in Form einer wäßrigen Lösung, und
      . das Ansäuerungsmittel;

      und dies solange, bis die gewünschte Menge von Siliciumdioxid gebildet ist,
      wobei das Reaktionsmedium bei dieser zweiten Stufe einen etwa konstanten pH-Wert in der Größenordnung von 8 bis 10 aufweist und auf einer Temperatur in der Größenordnung von 80 °C bis 98 °C gehalten wird,

**dadurch gekennzeichnet**, daß

   * der in der ersten Stufe eingesetzte Ausgangsansatz ein Elektrolyt-Salz aus der Gruppe der Alkalimetalle umfaßt, wobei die Menge an Elektrolyt mindestens etwa 4 mol beträgt und vorzugsweise in der Größenordnung von 0,4 bis 1,5 mol Alkalimetall-Ionen pro Liter Ansatz liegt,
   * die in der zweiten Stufe eingesetzte Lösung des Alkalimetallsilicates mindestens etwa 100 g $SiO_2$/Liter, vorzugsweise in der Größenordnung von 100 bis 330 g $SiO_2$/Liter enthält,
   * und dadurch, daß die genannte zweite Stufe unter solchen Bedingungen realisiert wird, daß die Kinetik C der Bildung von aktivem Siliciumdioxid, ausgedrückt in Gramm Siliciumdioxid/Stunde/Gramm Träger dem folgenden Wert entspricht:

$$C \geq 3 \, (A/200) \, 2^n,$$

vorzugsweise $C \geq 4 \, (A/200) \, 2^n$, und ganz besonders bevorzugt $C \geq 6 \, (A/200) \, 2^n$, worin

   . $n$ gleich $(T-90)/10$ ist,
   . $A$ die spezifische Oberfläche des zu umhüllenden Trägers darstellt, ausgedrückt in $m^2/g$, und
   . $T$ die Temperatur in °C ist.

2. Verfahren nach Anspruch 1, **dadurch gekennzeichnet**, daß das Alkalimetallsilicat ein Silicat von Natrium oder Kalium ist.

3. Verfahren nach Anspruch 1 oder 2, **dadurch gekennzeichnet**, daß das Ansäuerungsmittel eine Mineralsäure oder organische Säure ist.

4. Verfahren nach Anspruch 3, **dadurch gekennzeichnet**, daß das Ansäuerungsmittel Schwefelsäure, Salpetersäure, Chlorwasserstoffsäure, Essigsäure, Ameisensäure oder Kohlensäure ist.

5. Verfahren nach Anspruch 4, **dadurch gekennzeichnet**, daß das Ansäuerungsmittel Schwefelsäure in Form einer wäßrigen Lösung ist, die eine Konzentration in der Größenordnung von 60 g/l bis 400 g/l aufweist.

6. Verfahren nach irgendeinem der vorstehenden Ansprüche, **dadurch gekennzeichnet**, daß das den Träger bildende Material unter den Materialien ausgewählt wird, die geeignet sind, durch eine der folgenden Operationen wieder entfernt zu werden, und zwar thermische Behandlung für feste oder flüssige Materialien, Behandlung durch Auflösung mit Hilfe einer Mineralsäure oder organischen Säure bei einem pH-Wert von unter 8, vorzugsweise in der Größenordnung von 2 bis 7, oder Behandlung mit Hilfe eines apolaren Lösungsmittels für feste Materialien oder Abtrennung für flüssige Materialien, und dadurch, daß das genannte Material durch Anwendung der entsprechenden Operation entfernt wird.

7. Verfahren nach Anspruch 6, **dadurch gekennzeichnet**, daß die festen Materialien, die geeignet sind, durch Auflösung mit Hilfe einer Säure entfernt zu werden, unter den anorganischen Salzen, den Metallen, den organischen Polymeren und den vernetzten Polysacchariden, die bei einem pH-Wert von unter 8, vorzugsweise in der Größenordnung von 2 bis 7 löslich sind, ausgewählt werden.

8. Verfahren nach Anspruch 7, **dadurch gekennzeichnet**, daß das genannte Material Calciumcarbonat ist.

9. Verfahren nach Anspruch 6, **dadurch gekennzeichnet**, daß die festen Materialien, die geeignet sind, durch Auflösung mit Hilfe eines apolaren Lösungsmittels entfernt zu werden, unter den organischen, nicht alkalilöslichen Polymeren ausgewählt werden.

10. Verfahren nach Anspruch 6, **dadurch gekennzeichnet**, daß die flüssigen Materialien, die geeignet sind, durch Abtrennung entfernt zu werden, unter den Pflanzenölen, den Mineralölen, Vaselineöl und den Siliconölen ausgewählt werden.

11. Verfahren nach Anspruch 10, **dadurch gekennzeichnet**, daß die Operation der Abtrennung eine Operation der Zentrifugierung, Filtration, Destillation, Verdampfung, Dialyse oder Osmose ist.

12. Verfahren nach irgendeinem der vorstehenden Ansprüche, **dadurch gekennzeichnet, daß** das Material, das anders ist als Siliciumdioxid, eine Größe im Bereich von 20 nm bis 30 $\mu$m, vorzugsweise im Bereich von 50 nm bis 20 $\mu$m aufweist.

13. Verfahren nach irgendeinem der vorstehenden Ansprüche, **dadurch gekennzeichnet, daß** der Elektrolyt Natriumsulfat, Natriumchlorid, Natriumnitrat oder Natriumhydrogencarbonat ist.

14. Verfahren nach irgendeinem der vorstehenden Ansprüche, b der Träger in Form einer wäßrigen Dispersion, wenn es sich um ein festes Material handelt, oder in Form einer wäßrigen Emulsion, wenn es sich um eine Flüssigkeit handelt, eingesetzt wird.

15. Verfahren nach irgendeinem der vorstehenden Ansprüche, **dadurch gekennzeichnet, daß** die Menge an Träger, der eingesetzt werden kann, so beschaffen ist, daß der gebildete Ansatz in der Größenordnung von mindestens 10 % seines Gewichtes festen Träger oder in der Größenordnung von mindestens 10 % seines Volumens Träger in Form von Flüssigkeit enthält.

16. Verfahren nach Anspruch 15, **dadurch gekennzeichnet**, daß der Ansatz bis zu 50 % seines Gewichtes oder seines Volumens festen oder flüssigen Träger enthält.

17. Verfahren nach irgendeinem der vorstehenden Ansprüche, **dadurch gekennzeichnet, daß** die zweite Stufe zur Bildung der Aufschlämmung durch gleichzeitiges Eintragen von Alkalimetallsilicat und Ansäuerungsmittel realisiert wird, bis zur Bildung von mindestens 1 bis 150 Gewichtsteilen $SiO_2$ pro 100 Gewichtsteile Träger.

18. Verwendung der hohlen Teilchen von Siliciumdioxid, erhalten nach dem Verfahren, das den Gegenstand von irgendeinem der vorstehenden Ansprüche bildet, als Material oder Bestandteil von thermisch oder schallisolierenden Materialien, als hohle Füllstoffe für Polymere, Baustoffe, Kautschuk, Papier, Anstrichstoffe, als absorbierende Mittel, als Schleifmittel oder Zusatzstoffe für Zahnpasta, als Träger für die Absorption und/oder das Freisetzen von aktivem Material, wobei die Freisetzung des genannten aktiven Materials mittels Zerstörung der Schale aus Siliciumdioxid durch mechanisches Zerbrechen oder durch Auflösung der Schale aus Siliciumdioxid im basischen Medium oder durch Diffusion realisiert wird, als Sonnenschutzmittel oder Träger von Sonnenschutzmitteln oder zur Formulierung von flüssigen Stoffen in fester Form.

19. Spröde, hohle Teilchen von dichtem Siliciumdioxid, erhalten nach dem Verfahren, das den Gegenstand von irgendeinem der Ansprüche 1 bis 17 bildet, **dadurch gekennzeichnet**, daß sie aufweisen

- eine Oberfläche BET in der Größenordnung von 15 bis 800 m$^2$/g,
- eine Ölaufnahme DOP von höher als 500 ml/ 100 g Siliciumdioxid,
- eine Dicke der Schale in der Größenordnung von 2 bis weniger als 10 nm, und
- eine Granulometrie von über etwa 15 $\mu$m.

## Claims

1. Method for preparing hollow particles comprising a dense silica shell by precipitation of active silica from an aqueous alkali metal M silicate solution, with an $SiO_2/Na_2O$ ratio of at least 2, preferably of the order of 2.5 to 4, with adjustment of the pH using an acidifying agent, on a support made of a material other than silica, separation of the silica slurry formed and drying of the silica suspension recovered, with removal of the support without dissolution or destruction of the active silica shell, the operation of formation of silica slurry by precipitation being carried out according to the following stages:

   * a first stage consisting in employing an initial tank stock with a pH of the order of 8 to 10, comprising

      . water
      . at least one organic or inorganic support other than silica, which support is insoluble in water under the pH and temperature conditions of the slurry formation operation but which is capable of being at least partially removable without dissolution or destruction of the silica shell during the subsequent removal operation,
      . optionally a buffer or basic agent,

      at a temperature of the order of 80 to 98°C;
   * a second stage consisting in introducing, into the said tank stock,

      · the alkali metal silicate in the form of an aqueous solution,
      · and the acidifying agent,

      and in doing so until the desired amount of silica is formed,
      the reaction medium in this second stage having a substantially constant pH of the order of 8 to 10 and being maintained at a temperature of the order 80 to 98°C,
      the said process being **characterized in that**
   * the initial tank stock used in the first stage comprises an electrolytic salt of the group of alkali metals, the amount of electrolyte present being at least about 0.4 mol and preferably of the order of 0.4 to 1.5 mol of alkali metal ion per litre of tank stock,
   * the alkali metal silicate solution used in the second stage contains at least about 100 grams of $SiO_2$/litre, and preferably of the order of 100 to 330 grams of $SiO_2$/litre,
   * and in that the said second stage is carried out under conditions such that the kinetics K of formation of active silica, expressed in grams of silica/hour/gram of support, corresponds to a value

$$K \geq 3 \, (A/200) \, 2^n,$$

preferably $K \geq 4 \, (A/200) \, 2^n$
and very particularly $K \geq 6 \, (A/200) \, 2^n$

   . $n$ being equal to $(T-90)/10$
   . $A$ representing the specific surface, expressed in $m^2/g$, of the support to be coated
   . and T the temperature in °C.

2. Method according to Claim 1, **characterized in that** the alkali metal silicate is a sodium or potassium silicate.

3. Method according to Claim 1 or 2, **characterized in that** the acidifying agent is an inorganic or organic acid.

4. Method according to Claim 3, **characterized in that** the acidifying agent is sulphuric, nitric, hydrochloric, acetic, formic or carbonic acid.

5. Method according to Claim 4, **characterized in that** the acidifying agent is sulphuric acid, in the form of an aqueous solution exhibiting a concentration of the order of 60 to 400 g/l.

6. Method according to any one of the preceding claims, **characterized in that** the material constituting the support is chosen from materials capable of being removed by one of the following operations: heat treatment for solid or liquid materials, treatment by dissolution using an inorganic or organic acid at a pH of less than 8, preferably of the order of 2 to 7, or using a non-polar solvent for solid materials, or separation for liquid materials, and in that the said material is removed by employing the corresponding operation.

7. Method according to Claim 6, **characterized in that** the solid materials capable of being removed by dissolution using an acid are chosen from inorganic salts, metals, organic polymers or crosslinked polysaccharides which are soluble at a pH of less than 8, preferably of the order of 2 to 7.

8. Method according to Claim 7, **characterized in that** the said material is calcium carbonate.

9. Method according to Claim 6, **characterized in that** the solid materials capable of being removed by dissolution using a non-polar solvent are chosen from non-alkali-soluble organic polymers.

**10.** Method according to Claim 6, **characterized in that** the liquid materials capable of being removed by separation are chosen from vegetable oils, mineral oils, liquid petrolatum or silicone oils.

**11.** Method according to Claim 10, **characterized in that** the separation operation is a centrifuging, filtration, distillation, evaporation, dialysis or osmosis operation.

**12.** Method according to any one of the preceding claims, **characterized in that** the material other than silica exhibits a size of the order of 20 nm to 30 μm, preferably of the order of 50 nm to 20 μm.

**13.** Method according to any one of the preceding claims, **characterized in that** the electrolyte is sodium sulphate, chloride, nitrate or hydrogencarbonate.

**14.** Method according to any one of the preceding claims, **characterized in that** the support is employed in the form of an aqueous dispersion, when it is a solid material, or in the form of an aqueous emulsion, if it is a liquid.

**15.** Method according to any one of the preceding claims, **characterized in that** the amount of support which can be employed is such that the tank stock formed contains of the order of at least 10% of its weight of solid support or of the order of at least 10% of its volume of support in liquid form.

**16.** Method according to Claim 15, **characterized in that** the tank stock contains up to 50% of its weight or of its volume of solid or liquid support.

**17.** Method according to any one of the preceding claims, **characterized in that** the second stage of slurry formation is carried out by simultaneous introduction of alkali metal silicate and of acidifying agent, until the formation of at least 1 to 150 parts by weight of $SiO_2$ per 100 parts by weight of support.

**18.** Use of the hollow silica particles obtained according to the method forming the subject-matter of any one of the preceding claims as thermal or sound insulating materials or constituents thereof, as hollow fillers for polymers, building materials, rubber, paper or paint, as absorbing agents, as dentifrice additives or abrasives, as supports for the absorption and/or the release of active material, the release of the said active material being achieved by destruction of the silica shell by mechanical rupture or by dissolution in basic medium of the silica gel or by diffusion, as sun protection agents or sun protection agent support, or for the formulation in solid form of liquid materials.

**19.** Brittle hollow particles of dense silica obtained according to the method which is the subject of any one of Claims 1 to 17, **characterized in that** they exhibit

- a BET [lacuna] surface of the order of 15 to 800 $m^2/g$
- a DOP oil uptake of greater than 500 ml/100 g of silica
- and a shell thickness of the order of 2 to less than 10 nm.
- and a particle size of greater than approximately 15 μm.